# EUROPÄISCHE PATENTSCHRIFT

(11) **EP 1 512 462 B1**
(45) Veröffentlichungstag und Bekanntmachung des Hinweises auf die Patenterteilung: **09.12.2009**
(21) Anmeldenummer: 04020921.5
(22) Anmeldetag: 02.09.2004
(51) Int. Cl.: B01L 3/00

(54) **Abdeckung einer Hybridisierungskammer**
Cover for hybidization chamber
Couvercle de chambre d'hybridation

(30) Priorität: 03.09.2003 DE 10340473
(43) Veröffentlichungstag der Anmeldung: 09.03.2005
(73) Patentinhaber: EPPENDORF AG, 22339 Hamburg (DE)
(72) Erfinder: Hamels, Sandrine, 1474 Ways (BE); Van Huffel, Christophe, 1170 Brussel (BE); Seippel, Martin, 23758 Oldenburg (DE)
(74) Vertreter: Hauck Patent- und Rechtsanwälte

(56) Entgegenhaltungen:
- EP-A- 0 733 714
- WO-A-01/44515
- US-A- 6 037 168
- US-A1- 2003 109 059
- US-B1- 6 258 593

## Beschreibung

Die Erfindung bezieht sich auf eine Abdeckung einer Hybridisierungskammer.

Die Nukleinsäure-Hybridisierung ermöglicht die Feststellung des Vorhandenseins oder der Position einer bestimmten Nukleinsäuresequenz in einer Molekülgemischlösung oder einem histologischen Schnitt. Sie hat vielfältige Einsatzmöglichkeiten in der Forschung, z.B. der Genexpressionsanalyse oder der Bestimmung von Punktmutationen (SNP Single Nucleotide Polymorphism). Ferner findet sie Verwendung in der Diagnose erblicher Krankheiten oder einer diesbezüglichen Disposition, der Personenidentifizierung oder der Erforschung von Verwandtschaftsverhältnissen.

Bei der Hybridisierung wird von dem Phänomen Gebrauch gemacht, dass zwei zueinander komplementäre Nukleinsäure-Einzelstränge in Lösung beim Aufeinandertreffen unter bestimmten Puffer- und Temperaturbedingungen einen Doppelstrang bilden. Diese Doppelstrang-Bildung nennt man Hybridisierung.

Für die Hybridisierung werden einzelsträngige Nukleinsäure-Fragmente an einer festen Phase immobilisiert. Meist ist die Basenabfolge (Sequenz) der immobilisierten Nukleinsäure bekannt. Laut Übereinkunft [Phimister, Nature Genetics 21, 1-1 (1999)] wird die immobilisierte Nukleinsäure als englisch "Probe" (Sonde) bezeichnet.

Die zu untersuchende Nukleinsäure wird nach der selben Nomenklatur als englisch "Target" (Ziel-Nukleinsäure) bezeichnet. Sie wird in der Regel mit einem Label markiert, das einen Nachweis ermöglicht. Meist werden als Label Fluoreszenzfarbstoffe oder Radioisotope eingesetzt. Aber auch Label, wie z.B. Metalle (Gold, Silber, Platin) oder Enzyme werden verwendet.

Bei der Chip-Technologie wird nicht markierte Nukleinsäure in Form von punktförmigen Bereichen ("Spots") auf einem Träger fixiert. In der Regel ist der Träger ein Objektträger ("Slide") ,wie er aus der Mikroskopie bekannt ist. Auch andere Materialien kommen als feste Phase zum Einsatz, vorzugsweise hoch transparente Kunststoffe oder Silizium Chips.

Der Durchmesser der Spots liegt meist im Bereich von wenigen µm bis zu Hunderten µm. Die Dichte der Spots variiert von wenigen Spots pro Slide bis hin zu einigen Hunderttausend Stück pro cm². Dabei werden die Spots in einem genau festgelegten Muster ("Microarray") angeordnet. Danach wird eine Lösung mit den Target-Molekülen auf das Microarray aufgebracht. Die Hybridisierung erfolgt für die Dauer von einigen Minuten bis zu vielen Stunden unter der Zufuhr von Wärme. Danach wird die Hybridisierungslösung entfernt und der Träger gewaschen. Anschließend wird durch einen quantitativen Nachweis der Label festgestellt, ob eine Bindung der markierten Nukleinsäure an bestimmten Zielfeldern auf dem Chip erfolgt ist.

Statt in Spots angeordneter Microarrays werden auch histologische Schnitte durch "in situ" Hybridisierung auf derartigen Trägern untersucht. Dazu werden Gewebeschnitte auf einem Objektträger übertragen und die darin enthaltenen Nukleinsäuren durch eine Fixierung in einzelsträngige Form überführt. Die Hybridisierung erfolgt analog, wie vorstehend für Microarrays beschrieben.

Eine weitere Verwendungsmöglichkeit für die hier vorgeschlagene Hybridisierungskammer sind die Inkubation von Protein Microarrays und von Gewebeschnitten mit Antikörperlösungen sowie anderer spezifisch bindender Moleküle.

Zur Durchführung der Hybridisierung ist es bekannt, das Mikroarray auf dem Träger mit einer Abdeckung zu versehen, in die Lösung mit den DNA bzw. RNA-Sonden hineingefüllt wird.

Eine bekannte Abdeckung besteht aus einer PC-Folie mit etwa 0,2 mm Wandstärke und einer haubenförmigen Erhöhung zum Ausbilden der Hybridisierungskammer. Der um die Erhöhung umlaufende Randbereich der Folie wird mittels doppelt wirkender Klebebänder mit dem Träger verklebt. Die zwischen Erhöhung und Träger gebildete Hybridisierungskammer ist über oberseitige Befüll- und Entlüftungslöcher befüll- und ggfs. entleerbar. Vielfach wird die Abdeckung mit der enthaltenen Flüssigkeit vom Träger abgenommen.

Nachteilig bei den bekannten Abdeckungen ist, daß das Befüllen und ggfs. Entleeren über die kleinen Löcher in der dünnwandigen Folie durch Ansetzen einer Pipettenspitze schwierig ist. Beim Befüllen kommt es leicht zum Einbringen von Blasen. Mit Blasen belegte Spots sind von der Hybridisierung ausgeschlossen. Außerdem kommt es im Verlauf der Hybridisierung leicht zu Undichtigkeiten zwischen Abdeckung und Träger. Dies besonders, wenn die Kammer zum Intensivieren der Hybridisierung und Verkürzen der Hybridisierungszeit geschüttelt wird. Hierdurch kann es zu Flüssigkeitsverlust und Lufteinzug kommen mit nachteiligen Auswirkungen auf die Hybridisierung. Auch das Ablösen der steifen Abdeckung vor dem Abspülen des Trägers ist problematisch. Hierbei kann der Träger aufgrund von Biegekräften brechen.

Davon ausgehend liegt der Erfindung die Aufgabe zugrunde, eine Abdeckung einer Hybridisierungskammer zur Verfügung zu stellen, die günstigere Anwendungseigenschaften aufweist.

Druckschrift US 6,258,593 B1 zeigt eine Hybridisierungskammer, bei der ein Substrat in eine Grundplatte eingesetzt und von einer Abdeckung abgedeckt wird. Die Abdeckung hat einen umlaufenden Rahmen bzw. eine Lippe und eine Deckwand, die an der Oberseite bündig mit dem Rahmen abschließt. Innerhalb der Deckwand sind Befüll- und Entlüftungslöcher angeordnet. Auf der Oberseite der Deckwand befindet sich oberhalb jedes Befüll- und Entlüftungslochs jeweils ein rohrförmiger Abschnitt, der eine Einrichtung zum Befüllen bzw. Entlüften der Hybridisierungskammer aufnehmen kann. Um die Abdeckung auf dem Substrat zu fixieren, wird außerhalb der rohrförmigen Abschnitte ein zusätzlicher Rahmen angeordnet, über den die Abdeckung mit Hilfe einer Anpreßplatte, die mit der Grundplatte verschraubt wird, an das Substrat gepreßt wird.

Davon ausgehend ist es die objektive technische Aufgabe der Erfindung, eine Abdeckung mit Befüll- und Entlüftungslöchern für eine Hybridisierungskammer anzugeben, die einfacher auf einem Substrat fixiert werden kann.

Die Aufgabe wird von einer Abdeckung mit den Merkmalen des Anspruches 1 gelöst. Vorteilhafte Ausgestaltungen der Abdeckung sind in den Unteransprüchen angegeben.

Die erfindungsgemäße Abdeckung einer Hybridisierungskammer hat
- einen umlaufenden Rahmen mit innerhalb des Rahmens von der Oberseite zur Unterseite des Rahmens verlaufenden Befüll- und Entlüftungslöchern,
- einen außerhalb der Befüll- und Entlüftungslöcher an der Unterseite des Rahmens umlaufenden Absatz,
- eine innerhalb des Rahmens angeordnete, mit seiner Unterseite im wesentlichen bündige und bezüglich seiner Oberseite nach unten versetzte Deckwand und
- besteht im wesentlichen aus mindestens einem elastischen Kunststoff.

Die erfindungsgemäße Abdeckung ist durch Verkleben des umlaufenden, von der Unterseite des Rahmens vorstehenden Absatzes mit der Oberseite eines Trägers eines Mikroarrays oder von Gewebeabschnitten verbindbar. Hierzu wird beispielsweise ein Kleber direkt appliziert oder ein Klebeband eingesetzt. Beim Ankleben des Rahmens ist die Oberseite vorteilhaft für das Ansetzen eines Andrückwerkzeuges nutzbar. Z.B. ist der Rahmen durch Abrollen eines zylindrischen Werkzeuges anpreßbar. Die Andrückkräfte werden direkt in die darunter befindliche Verklebung eingeleitet.

Durch die Verbindung der Abdeckung mit dem Träger entsteht eine Hybridisierungskammer. Diese ist durch ein Befülloch mit Flüssigkeit befüllbar, beispielsweise durch Ansetzen einer Pipettenspitze. Der Rahmen hat eine größere Höhe als die Deckwand. Infolgedessen ist die Möglichkeit gegeben, ein verhältnismäßig tiefes (z.B. trichterförmiges) Befülloch zu bilden, in das eine Pipettenspitze gut abdichtend einsetzbar ist. Ein Lufteinzug beim Befüllen wird hierdurch vorteilhaft vermieden. Durch den im wesentlichen bündigen Abschluß der Deckwand mit der Unterseite wird das gleichmäßige und blasenfreie Befüllen des Hohlraumes zwischen Deckwand, Absatz und Träger mit Flüssigkeit begünstigt. Beim Befüllen entweicht die Luft aus dem Inneren der Hybridisierungskammer durch ein Entlüftungsloch.

Die nach unten versetzte Deckwand hat eine größere Flexibilität als der Rahmen. Etwaige Flüssigkeitsverluste, z.B. durch Verdunstung oder Undichtigkeit, sind durch Auswölben der Deckwand zum Träger hin kompensierbar. Ein Lufteinzug wird hierdurch vermieden. Ggfs. ist zum Abziehen der Flüssigkeit aus der Hybridisierungskammer eine Pipettenspitze an ein Entlüftungsloch gut abdichtend absetzbar. Die bezüglich der Oberseite des Rahmens nach unten versetzte Deckwand begünstigt die Elastizität der gesamten Abdeckung. Infolgedessen ist diese einfacher ohne Einleitung zerstörerischer Kräfte in dem Träger von diesem abziehbar.

Um Pipettenspitzen gut abdichtend an die Befüll- und Entlüftungslöcher anzusetzen, hat der Rahmen gemäß einer Ausgestaltung eine Höhe von mindestens etwa 1,5 bis 2 mm. Für eine elastische Ausbildung hat die Deckwand gemäß einer Ausgestaltung eine Wandstärke von maximal etwa 1 bis 2 mm.

Gemäß einer Ausgestaltung ist der Rahmen an der Oberseite und/oder Unterseite flach und/oder ist seine Außenseite und/oder Innenseite zu seiner Oberseite hin geneigt. Hierdurch wird das abdichtende Ankleben auf dem Träger und die Flexibilität der Abdeckung unterstützt.

Auf die Oberseite des Rahmens kann während der Hybridisierung zusätzlich Druck auf den Rahmen ausgeübt werden, der die Dichtigkeit der Klebeverbindung unterstützt. Dies ist besonders vorteilhaft bei einer ebenen, zur Unterseite planparallelen Oberseite des Rahmens möglich. Der Druck kann z.B. über eine Platte auf den Rahmen ausgeübt werden.

Gemäß einer Ausgestaltung ist der Rahmen im wesentlichen rechteckig und weist die Befüll- und Entlüftungslöcher an diagonal einander gegenüberliegenden Ecken auf. Hierdurch wird das gleichmäßige Befüllen, Entlüften und ggfs. Entleeren der Hybridisierungskammer gefördert. Gemäß einer Ausgestaltung hat der Rahmen an den Befüll- und Entlüftungslöcher aufweisenden Ecken nach innen gerichtete Ausbuchtungen. Dies begünstigt den Befüll- und ggfs. Entleerungsvorgang und die Ausbildung eines Absatzes mit einer für die Verklebung mit dem Träger vorteilhaften Breite außen um die Befüll- und Entlüftungslöcher.

Gemäß einer Ausgestaltung ist der Absatz im wesentlichen streifenförmig für eine flächige und dichte Verklebung mit dem Träger.

Gemäß einer Ausgestaltung ist der Absatz bogenförmig um die Befüll- und Entlüftungslöcher herumgeführt und dazwischen geradlinig. Gemäß einer Ausgestaltung erweitern sich die Befüll- und Entlüftungslöcher zumindest in einen Abschnitt zur Oberseite des Rahmens hin, wodurch eine vorteilhafte Anlage- und Abdichtfläche für eine Pipettenspitze gegeben ist. Hierfür weisen die Befüll- und Entlüftungslöcher gemäß einer weiteren Ausgestaltung eine an das Ende der Pipettenspitze angepaßte Aufnahme auf.

Gemäß einer Ausgestaltung ist an einer Außenseite des Rahmens eine parallel zur Rahmenebene vorstehende Lasche vorhanden. Die Lasche ist insbesondere für das korrekte Ausrichten der Abdeckung bezüglich eine Mikroarrays auf einem Träger von Vorteil und für das Abziehen der Abdeckung vom Träger. Hierfür ist gemäß einer weiteren Ausgestaltung die Lasche unten bündig mit der Unterseite des Rahmens.

Gemäß einer Ausgestaltung weist der Rahmen an einer Außenseite einen Versprung auf. Der Versprung begünstigt die platzsparende Anordnung mehrerer identisch ausgebildeter Abdeckungen nebeneinander mit einander zugewandtem Versprung. Ferner ist die Ausbildung des Anspritzpunktes im Bereich des Versprunges vorteilhaft.

Gemäß einer Ausgestaltung ist die Abdeckung im Bereich der Deckwand und/oder des Rahmens transparent. Dies begünstigt eine visuelle Beobachtung der Befüll- und ggfs. Entleerungsvorgänge. Hierfür kommt ein transparenter Kunststoff zum Einsatz. Gemäß einer weiteren Ausgestaltung ist zumindest der Bereich der Deckwand glasklar. Dies wird z.B. durch Einsatz von Spritzgießwerkzeugen mit hochpolierten Oberflächen erreicht.

Gemäß einer Ausgestaltung ist die Abdeckung aus einem TPU und/oder aus einem Weich-PVC hergestellt. Im übrigen sind Materialien einsetzbar, die eine oder mehrere der nachfolgenden Eigenschaften aufweisen: Elastizität, Klebetauglichkeit, Transparenz, Spritzbarkeit, möglichst geringe Bindung von und/oder Freisetzung von DNA bzw. RNA und/oder fluoreszierender Stoffe.

Die Abdeckung ist grundsätzlich aus mehreren elastischen Kunststoffen herstellbar. Gemäß einer Ausgestaltung ist sie einteilig aus einem elastischen Kunststoff hergestellt. Die Herstellung kann in einem oder mehreren Spritzschritten erfolgen. Vorteilhaft ist die Abdeckung in einem einzigen Spritzschritt einteilig herstellbar.

Gemäß einer Ausgestaltung umfaßt eine Hybridisierungskammer einen Träger, auf dem Träger immobilisiertes Biomaterial und mindestens eine auf dem Träger befestigte Abdeckung nach einem der Ansprüche 1 bis 18. Bei dem Biomaterial handelt es sich z.B. um Mikroarrays oder um Gewebeschnitte. Gemäß einer Ausgestaltung ist zwischen Träger und Abdeckung eine Verklebung vorhanden. Die Verklebung ist z.B. von einem Klebstoffauftrag oder einem doppelseitigen Klebeband gebildet.

Nachfolgend wird die Erfindung anhand der anliegenden Zeichnung eines Ausführungsbeispieles näher erläutert. In der Zeichnung zeigen:
- Fig. 1a bis j: die Abdeckung in der Draufsicht (Fig. 1a), in einer Seitenansicht (Fig. 1b), in einer Rückansicht (Fig. 1c), in einem Schnitt entlang der Linie A-A der Fig. 1a (Fig. 1d), in einem Schnitt entlang der Linie A-A der Fig. 1a (Fig. 1e), in einem Schnitt entlang der Linie C-C der Fig. 1a (Fig. 1f), in einer vergrößerten Einzelheit D der Fig. 1e (Fig. 1g), in einer Perspektivansicht schräg von oben (Fig. 1h), in einer Unteransicht (Fig. 1i) und in einer Perspektivansicht schräg von unten (Fig. 1j);
- Fig. 2a und b: zwei Kammern gemäß Fig. 1 auf einem Träger in einer perspektivischen Ansicht schräg von oben (Fig. 2a) und einer verkleinerten Draufsicht (Fig. 2b);
- Fig. 3a und b: zwei Kammern gemäß Fig. 1 auf einem Träger in einer weiteren perspektivischen Ansicht schräg von oben (Fig. 3a) und in einer Perspektiven schräg von oben von der anderen Seite (Fig. 3b).

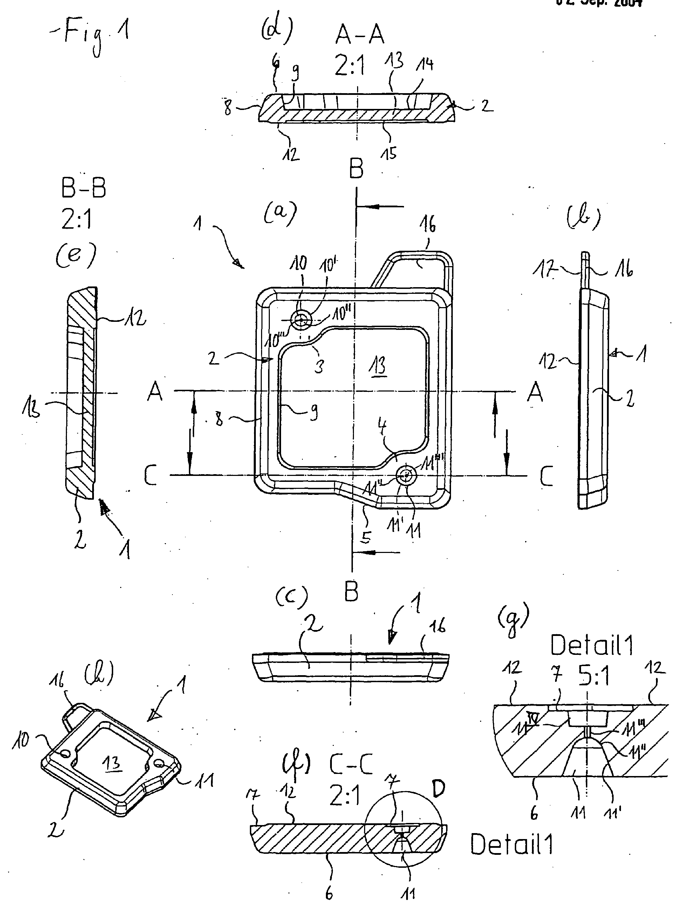

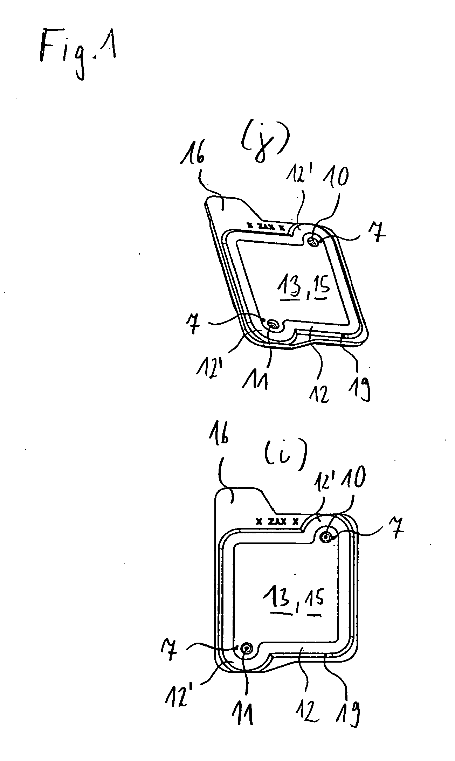

Gemäß Fig. 1 hat eine Abdeckung 1 einen umlaufenden Rahmen 2, der im wesentlichen rechteckförmig ist. An diagonal einander gegenüberliegenden Ecken hat der Rahmen 2 nach innen gerichtete Ausbuchtungen 3, 4. An der Ecke mit der Ausbuchtung 4 hat der Rahmen 2 an der Außenseite einen Versprung 5. Hier sind zwei parallele Abschnitte derselben Außenseite gegeneinander versetzt, wobei sie im Mittelbereich dieser Außenseite durch einen angewinkelten Abschnitt miteinander verbunden sind.

Der Rahmen 2 hat eine flache Oberseite 6 und eine dazu parallele, flache Unterseite 7. Ferner weist er umlaufend eine Außenseite 8 und eine Innenseite 9 auf, die zur Oberseite 6 hin geneigt sind.

In den Ecken mit den Ausbuchtungen 3, 4 hat der Rahmen 2 Befüll- und Entlüftungslöcher 10, 11, welche von der Oberseite 6 zur Unterseite 7 des Rahmens hin verlaufen. Die Befüll- und Entlüftungslöcher 10, 11 haben angrenzend an die Oberseite 6 einen konischen Abschnitt 10', 11' mit einem napfförmigen Abschnitt 10", 11". Daran schließt sich ein röhrchenförmiger Verbindungsabschnitt 10"', 11"' an. Der röhrchenförmige Verbindungsabschnitt 10"', 11'" ist auf der anderen Seite mit einem leicht konischer Endabschnitt 10^{IV} (nicht gezeigt), 11^{IV} verbunden, der sich zur Unterseite 7 hin erweitert, in der er mündet.

Der Rahmen 2 hat an der Unterseite 7 einen unten vorstehenden, streifenförmigen Absatz 12, der um den gesamten Rahmen 2 umläuft. Der Absatz 12 verläuft entlang der Innenseiten des Rahmens 2. Um die Befüll- und Entlüftungslöcher 10, 11 ist er außen bogenförmig herumgeführt. Innerhalb dieser Bögen 12' erstreckt sich die Unterseite 7 innerhalb des bogenförmig herumgeführten Absatzes 12 um die Befüll- und Entlüftungslöcher 10, 11 bis zur Innenseite des Rahmens 2 im Bereich der Auswölbungen 3, 4. Die Breite des Absatzes 12 entspricht etwa der halben Breite (im Beispiel etwas mehr) der Unterseite 7.

Die konischen Abschnitte 10', 11' und napfförmigen Abschnitte 10", 11" sind so bemessen, daß das Ende einer handelsüblichen Pipettenspitze einsetzbar ist, so daß sie sicher seitlich geführt und abgedichtet ist.

Ferner hat die Abdeckung 1 eine Deckwand 13, deren Oberseite 14 bezüglich der Oberseite 6 des Rahmens 2 nach unten versetzt ist, im Beispiel auf weniger als die halbe Höhe des Rahmens 2, gemessen zwischen seiner Oberseite 6 und seiner Unterseite 7. Die Deckwand 13 hat eine Unterseite 15, die mit der Unterseite 7 des Rahmens fluchtet. In den Bereichen der Ausbuchtungen 3, 4 gehen die Unterseite der Deckwand 15 und die Unterseite 7 des Rahmens 2 ineinander über.

Schließlich hat die Abdeckung 1 an der Außenseite des Rahmens 2, die der Außenseite mit dem Versprung 5 gegenüberliegt, eine nach außen vorstehende Lasche 16. Die Lasche 16 ist in der Draufsicht trapezförmig. Ihre Unterseite 17 ist bündig mit der Unterseite 7 des Rahmens 2.

Im Beispiel hat die von den Abschnitten 10', 11', 10", 11" gebildete Aufnahme für eine Pipettenspitze eine Tiefe von etwa 2 mm. Dementsprechend beträgt die Höhe des Rahmens etwa 4 mm und die Wandstärke der Deckwand 13 etwa 1 bis 2 mm.

Die Außenabmessungen des Rahmens 2 betragen an den Seiten mit der Lasche 16 und dem Versprung 5 maximal etwa 35 mm und quer dazu etwa 25 mm.

Die Abdeckung 1 ist im Beispiel einteilig aus einem thermoplastischen Polyurethan (TPU) in einem einzigen Schritt gespritzt.

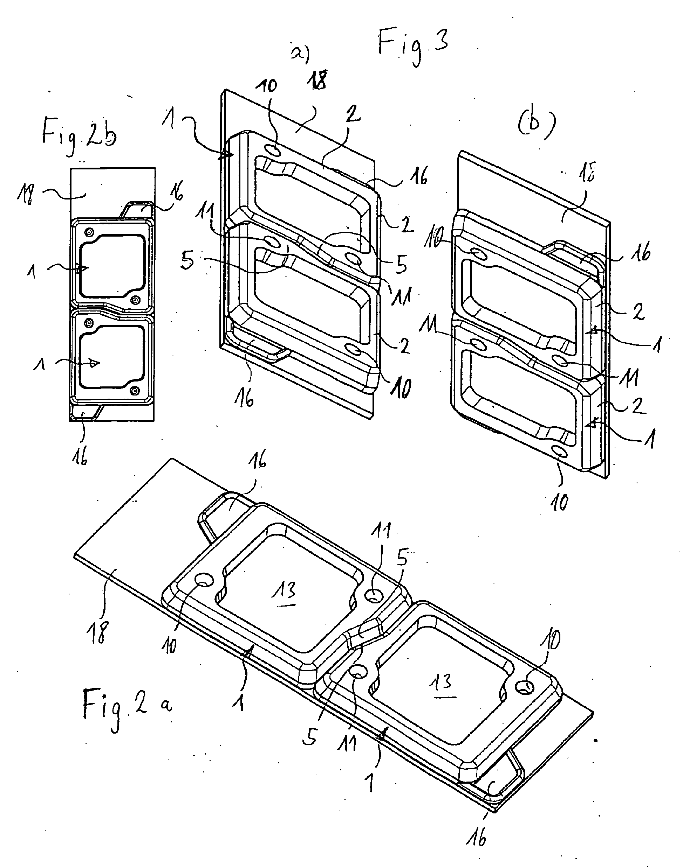

Gemäß Fig. 2 sind zwei Abdeckungen 1 der vorbeschriebenen Art auf einem Träger 18 aus Glas fixiert. Es handelt sich dabei um eine rechteckige Scheibe mit Abmessungen von 25 x 75 mm. In diesen Abmessungen sind Slides weit verbreitet.

Die beiden Abdeckungen 1 sind mit den langen Außenseiten ihres Rahmens 2 bündig an die langen Außenseiten des Slides 18 angesetzt. Ferner ist eine Abdeckung 1 mit ihrer Lasche 16 bündig an eine kurze Außenseite des Slides 18 angesetzt. Beide Abdeckungen 1 sind mit ihren Versprüngen 5 aneinandergesetzt. Durch diese Anordnung wird eine genaue Positionierung der Abdeckungen 1 bezüglich des Slides 18 erreicht. Infolgedessen befinden sich zuvor auf den Slides 18 aufgebrachte Mikroarrays genau unter dem Bereich der Deckwände 13 der beiden Abdeckungen 1.

Die Abdeckungen 1 sind z.B. mittels doppelseitiger Klebebänder auf dem Slide 18 aufgeklebt. Diese können bereits auf die Abdeckungen 1 vorgeklebt sein. Es handelt sich hierbei bevorzugt um Klebebänder, die zur Abdeckung 1 und zum Slide 18 hin mit verschiedenen Klebstoffen versehen sind, die eine optimale Verklebung zur Abdeckung 1 und zum Slide 18 hin gewährleisten, ohne die Durchführung der Hybridisierung zu beeinträchtigen. Geeignet ist z.B. ein Kleber auf Acrylatbasis zur Abdeckung hin und ein silikonisierter Kleber zum Glas hin.

Der Absatz 12 hat außen eine Fase 19 (vgl. Fig. 1i und j). Die Fase 19 begünstigt das Heraustreten überschüssigen Klebstoffes, z.B. bei Verkleben mittels eines Klebebandes oder mittels eines gesondert aufgebrachten Klebstoffes.

Die von den Abdeckungen 1 und dem Slide 18 gebildeten Hybridisierungskammern werden durch Ansetzen der Pipettenspitze einer Pipettiervorrichtung an die Befüllöcher 10 oder 11 durch Hineindrücken der Flüssigkeit befüllt. Dabei wird die Luft aus der Hybridisierungskammer durch die Entlüftungslöcher 11 oder 10 verdrängt, so daß der gesamte Hohlraum zwischen der Deckwand 13 und dem Träger 18 blasenfrei mit Flüssigkeit befüllt wird. Die etwaige Verdunstung von Flüssigkeit während der mehrere Stunden dauernden Hybridisierung wird durch die Nachgiebigkeit der Deckwände 13 kompensiert, die sich nach innen leicht auswölben. Auch wird durch ein Vibrieren der Deckwände 13 bei einem Schütteln die Vermischung der Flüssigkeit und damit der Vorgang der Hybridisierung intensiviert.

Nach Abschluß der Hybridisierung kann die Flüssigkeit durch eines der Befüll- und Entlüftungslöcher 10, 11 mittels einer Pipettenspitze abgesaugt werden. Die Flüssigkeit kann aber auch in der Abdeckung verbleiben. Schließlich werden die Abdeckungen 1 durch Ziehen an den Laschen 16 von dem Slide abgeschält, wobei sie sich in Richtung der angelegten Kraft ausbiegen.

## Patentansprüche

1. Abdeckung einer Hybridisierungskammer, die im Wesentlichen aus einem Kunststoff besteht, mit
- Befüll- und Entlüftungslöchern (10, 11),
- einem umlaufenden Rahmen (2) mit einem außerhalb der Befüll- und Entlüftungslöcher (10, 11) an der Unterseite (7) des Rahmens umlaufenden Absatz (12) und
- einer innerhalb des Rahmens (2) angeordneten Deckwand (13),
**dadurch gekennzeichnet, daß**
- die Deckwand (13) mit der Unterseite (7) des Rahmens bündig und bezüglich der Oberseite (6) des Rahmens nach unten versetzt ist,
- die Befüll- und Entlüftungslöcher (10, 11) innerhalb des Rahmens von der Oberseite (6) zur Unterseite (7) des Rahmens verlaufen und
- der Kunststoff elastisch ist.

2. Abdeckung nach Anspruch 1, bei der der Rahmen eine Höhe von mindestens etwa 1,5 bis 2 mm und/oder bei der die Deckwand (13) eine Wandstärke von maximal etwa 1 bis 2 mm aufweist.

3. Abdeckung nach Anspruch 1 oder 2, bei der die Oberseite (6) und/oder Unterseite (7) des Rahmens (2) flach ist und/oder die Außenseite (8) und/oder die Innenseite (9) des Rahmens (2) zu seiner Oberseite (6) hin geneigt ist.

4. Abdeckung nach einem der Ansprüche 1 bis 3, deren Rahmen im wesentlichen rechteckig ist und die Befüll- und Entlüftungslöcher (10, 11) an diagonal einander gegenüberliegenden Ecken aufweist.

5. Abdeckung nach einem der Ansprüche 1 bis 4, deren Rahmen (2) an den Befüll- und Entlüftungslöcher (10, 11) aufweisenden Ecken nach innen gerichtete Ausbuchtungen (3, 4) aufweist.

6. Abdeckung nach einem der Ansprüche 1 bis 5, deren Absatz (12) im wesentlichen streifenförmig ist.

7. Abdeckung nach einem der Ansprüche 1 bis 6, deren Absatz (12) bogenförmig um die Befüll- und Entlüftungsöffnungen (10, 11) herumgeführt und dazwischen geradlinig ist.

8. Abdeckung nach einem der Ansprüche 1 bis 7, deren Befüll- und Entlüftungslöcher (10,11) sich zunächst in einem Abschnitt (10', 10", 11' 11") zur Oberseite (6) des Rahmens (2) hin erweitern.

9. Abdeckung nach einem der Ansprüche 1 bis 8, deren Befüll- und Entlüftungslöcher (10,11) eine an das Ende einer Pipettenspitze angepaßte Aufnahme (10', 10", 11',11") aufweisen.

10. Abdeckung nach einem der Ansprüche 1 bis 9, die an einer Außenseite des Rahmens (2) eine parallel zur Rahmenebene vorstehende Lasche (16) aufweist.

11. Abdeckung nach Anspruch 10, deren Lasche (16) unten bündig mit der Unterseite (7) des Rahmens (2) ist.

12. Abdeckung nach einem der Ansprüche 1 bis 11, deren Rahmen (2) an einer Außenseite einen Versprung (5) aufweist.

13. Abdeckung nach einem der Ansprüche 1 bis 12, die im Bereich der Deckwand (13) und/oder des Rahmens (2) transparent ist.

14. Abdeckung nach einem der Ansprüche 1 bis 13, die zumindest im Bereich der Deckwand (13) glasklar ist.

15. Abdeckung nach einem der Ansprüche 1 bis 14, die aus einem TPU und/oder aus einem Weich-PVC hergestellt ist.

16. Abdeckung nach einem der Ansprüche 1 bis 15, die einteilig aus einem elastischen Kunststoff hergestellt ist.

17. Abdeckung nach einem der Ansprüche 1 bis 16, deren Absatz (12) unten mit einem doppelseitigen Klebeband verbunden ist.

18. Abdeckung nach Anspruch 17, bei der das doppelseitige Klebeband zur Abdeckung hin einen Acrylatkleber und auf der gegenüberliegenden Seite einen silikonisierten Kleber aufweist.

19. Hybridisierungskammer mit einem Träger (18), auf dem Träger (18) immobilisiertem Biomaterial und mindestens einer auf dem Träger (18) befestigten Abdeckung (1) nach einem der Ansprüche 1 bis 18.

20. Hybridisierungskammer nach Anspruch 19, bei der zwischen Träger (18) und Abdeckung (1) eine Verklebung vorhanden ist.

## Claims

1. Cover of a hybridization chamber which substantially consists of a plastic material with
- filling and ventilation holes (10, 11),
- a peripheral frame (2) with a peripheral shoulder (12) outside the filling and ventilation holes (10, 11) on the lower face (7)
- a cover wall (13) arranged inside the frame (2),
**characterised in that**
- the cover wall (13) is flush with the lower face (7) of the frame and downwardly offset relative to the upper face (6) of the frame,
- the filling and ventilation holes (10, 11) extend from the upper face (6) to the lower face (7) of the frame within the frame and
- the plastic material is a resilient plastics material.

2. Cover according to claim 1, in which the frame has a height of at least approximately 1.5 to 2 mm and/or in which the cover wall (13) has a maximum wall thickness of approximately to 2 mm.

3. Cover according to claim 1 or 2, in which the upper face (6) and/or the lower face (7) of the frame (2) is flat and/or the outer face (8) and/or the inner face (9) of the frame (2) is inclined toward its upper face (6).

4. Cover according to any of claims 1 to 3, the frame thereof being substantially rectangular and comprising filling and ventilation holes (10, 11) at diagonally opposing corners.

5. Cover according to any of claims 1 to 4, the frame (2) thereof comprising inwardly facing bulges (3, 4) at the corners comprising filling and ventilation holes (10, 11).

6. Cover according to any of claims 1 to 5, the shoulder (12) thereof being substantially strip-shaped.

7. Cover according to any of claims 1 to 6, the shoulder (12) thereof being guided in a curved shape around the filling and ventilation holes (10, 11) and being rectilinear therebetween.

8. Cover according to any of claims 1 to 7, the filling and ventilation holes (10, 11) thereof then widening in a portion (10', 10", 11', 11") toward the upper face (6) of the frame (2).

9. Cover according to any of claims 1 to 8, the filling and ventilation holes (10, 11) thereof comprising a receiver (10', 10", 11', 11 ") adapted to the end of a pipette tip.

10. Cover according to any of claims 1 to 9, which comprises a tab (16) on an outer face of the frame (2) protruding parallel to the plane of the frame.

11. Cover according to claim 10, the tab (16) thereof being flush below with the lower face (7) of the frame (2).

12. Cover according to any of claims 1 to 11, the frame (2) thereof comprising an indentation (5) on an outer face.

13. Cover according to any of claims 1 to 12, which is transparent in the region of the cover wall (13) and/or the frame (2).

14. Cover according to any of claims 1 to 13, which at least in the region of the cover wall (13) is crystal clear.

15. Cover according to any of claims 1 to 14, which is manufactured from a thermoplastic polyurethane and/or from a soft PVC.

16. Cover according to any of claims 1 to 15, which is integrally manufactured from a resilient plastics material.

17. Cover according to any of claims 1 to 16, the underside of the shoulder (12) thereof being connected to a double-sided adhesive tape.

18. Cover according to claim 17 in which the double-sided adhesive tape comprises an acrylate adhesive toward the cover and a siliconised adhesive on the opposing face.

19. Hybridization chamber with a support (18), immobilised biomaterial on the support (18) and at least one cover (1) fastened onto the support (18) according to any of claims 1 to 18.

20. Hybridization chamber according to claim 19, in which a bond is present between the support (18) and the cover (1).

## Revendications

1. Couvercle de chambre de hybridation qui consiste essentiellement d'une matière plastique, avec
des trous pour remplissage et aération (10, 11),
un cadre circonférentiel (2) avec un épaulement (12) circulant dans le côté inférieur (7) du cadre au dehors des trous pour remplissage et aération (10, 11), et
une paroi de recouvrement (13) arrangée dans l'intérieur du cadre (2),
**caractérisé en ce que** la paroi de recouvrement (13) est à fleur du côté inférieur (7) du cadre, et décalée vers le bas du côté supérieur (6) du cadre,
dans le cadre, les trous pour remplissage et aération (10, 11) passent du côté supérieur (6) au côté inférieur (7) du cadre, et
la matière plastique est élastique.

2. Couvercle selon la revendication 1, dans lequel le cadre présente une hauteur d'au moins 1,5 à 2 mm environ, et/ou la paroi de recouvrement (13) une épaisseur de paroi maximale de 1 à 2 mm environ.

3. Couvercle selon la revendication 1 ou 3, dans lequel le côté supérieur (6) et/ou le côté inférieur du (7) du cadre (2) est plat, et/ou le côté extérieur (8) et/ou le côté intérieur (9) du cadre (2) est incliné vers son côté supérieur (6).

4. Couvercle selon l'une quelconque des revendications 1 à 3, dont le cadre (2) est essentiellement rectangulaire et présente les trous pour remplissage et aération (10, 11) sur des coins qui sont opposés l'un à l'autre de façon diagonale.

5. Couvercle selon l'une quelconque des revendications 1 à 4, dont le cadre (2) présente des courbures (3, 4) dirigées vers l'intérieur dans les coins qui présentent les trous pour remplissage et aération (10, 11).

6. Couvercle selon l'une quelconque des revendications 1 à 5, dont l'épaulement (12) est essentiellement en forme de bande.

7. Couvercle selon l'une quelconque des revendications 1 à 6, dont l'épaulement (12) est guidé en forme d'arc autour des trous pour remplissage et aération (10, 11), et est linéaire entre les deux.

8. Couvercle selon l'une quelconque des revendications 1 à 7, dont les trous pour remplissage et aération (10, 11) s'élargissent dans une partie (10', 10", 11', 11") tout près vers le côté supérieur (6) du cadre (2).

9. Couvercle selon l'une quelconque des revendications 1 à 8, dont les trous pour remplissage et aération (10, 11) présentent une accommodation (10', 10", 11', 11") adaptée à l'extrémité d'une pointe de pipette.

10. Couvercle selon l'une quelconque des revendications 1 à 9, qui présente une attache (16) se projetant en parallèle au plan du cadre sur un côté extérieur du cadre (2).

11. Couvercle selon la revendication 10, dont l'attache (16) est à fleur du côté inférieur (7) du cadre (2) au dessous.

12. Couvercle selon l'une quelconque des revendications 1 à 11, dont le cadre (2) présente une projection (5) dans un côté extérieur.

13. Couvercle selon l'une quelconque des revendications 1 à 12, qui est transparent dans la région de la paroi de recouvrement (13) et/ou du cadre (2).

14. Couvercle selon l'une quelconque des revendications 1 à 13, qui est clair comme le verre au moins dans la région de la paroi de recouvrement (13).

15. Couvercle selon l'une quelconque des revendications 1 à 14, qui est fait d'un TPU ou d'une PVC souple.

16. Couvercle selon l'une quelconque des revendications 1 à 15, qui est fait en une seule pièce d'une matière plastique élastique.

17. Couvercle selon l'une quelconque des revendications 1 à 16, dont l'épaulement (12) est raccordé au dessous par un ruban qui est adhésif aux deux côtés.

18. Couvercle selon la revendication 17, dans lequel le ruban qui est adhésif aux deux côtés présente une colle en acrylate dans son côté dirigé vers le couvercle, et une colle siliconée dans le côté opposé.

19. Chambre de hybridation avec un support (18), du matériau biologique immobilisé sur le support (18) et avec au moins un couvercle (1) selon l'une quelconque des revendications 1 à 18 fixé sur le support (18),

20. Chambre de hybridation selon la revendication 19, dans lequel un collage existe entre le support (18) et le couvercle (1).
